# EUROPEAN PATENT APPLICATION

(11) **EP 2 224 032 A1**
(43) Date of publication of application: **01.09.2010**
(21) Application number: 09152856.2
(22) Date of filing: 13.02.2009
(51) Int. Cl.: C22F 1/06, C22C 23/00, A61F 2/82

(54) **Process for manufacturing magnesium alloy based products**

(71) Applicant: Nederlandse Organisatie voor Toegepast -Natuurwetenschappelijk Onderzoek TNO, 2628 VK Delft (NL)
(72) Inventor: Müge, Erinc, 5641 GB Eindhoven (NL); Mannes, Raymond Gerardus Theodorus Marie, 6125 AJ Obbicht (NL); Sillekens, Wilhelmus Hubertina, 6049 HA Herlen (NL); Werkhoven, Robert Jan, 5673 LH Nuenen (NL)
(74) Representative: Hatzmann, Martin

(57) **Abstract**

A process for producing a magnesium based product, comprising the production of a feedstock out of a magnesium based alloy or a mixture of magnesium based alloys, a subsequent thermo-mechanical treatment of the feedstock, and an annealing step wherein the feedstock is produced by semi-solid processing. The invention further provides products, in particular implants and more particular stents made according to said process.

## Description

The present invention relates to a process for producing a magnesium based product, comprising the production of a feedstock out of a magnesium based alloy or a mixture of magnesium based alloys, a thermo-mechanical treatment of the feedstock, and an annealing step.

The present invention also relates to a product made by such a process.

### State of the art

Magnesium alloys are considered to be a new generation of biomedical implant materials. One of the advantages of magnesium alloys over other biocompatible metallic implant materials such as titanium alloys is that magnesium alloys have a stiffness that is close to that of the bone of human and animal beings. Another advantage is that magnesium alloys dissolve in the human and animal body through time (the alloys are biodegradable or bioresorbable) and therefore there is no need the remove the implants. Magnesium alloys can also be applied for automotive applications like drive train components and for other medical and non-medical applications where for instance weight reduction is an issue. However, the relative poor corrosion resistance of common magnesium alloys hinders the use for structural application in a non-protected or even hostile environment, including the human body with its corrosive body fluids. Metallic magnesium corrodes significantly at the physiological pH (acidity) value prevailing the human body, resulting in a loss of mechanical integrity of the implant. As a consequence the implant present in the human body loses its integrity before the healing process of the surrounding tissue is advanced sufficiently. For applications like stents and orthopaedic implants the materials should have not only a good corrosion resistance but also good mechanical properties, in particular a high ductility.

For practical applications pure magnesium often does not fulfil the requirements for a good processing or does not meet the demands set by the application of the product made out of the metal. For this reason magnesium is alloyed with elements that improve its properties. Such magnesium based alloys may contain for example rare-earth elements like Nd (Neodymium) and La (Lanthanum) or elements like Li (Lithium) and Mn (Manganese) for improving the corrosion resistance. To refine the grains of the materials, alloying elements like Y (Yttrium), Zr (Zirconium) and Sr (Strontium) are used. Elements like Ag (Silver), Cu (Copper), Zn (Zinc) and Cd (Cadmium) reduce the solid solution strengthening effect, whereas Sb (Antimony) can be used to improve tensile properties. It is appreciated that the effect of different alloying elements and their concentration on the properties of the magnesium based alloys can be found in the literature known to those skilled in the art (see e.g. Magnesium and Magnesium Alloys, ASM Specialty Handbook, editors: M.M. Avedesian and H. Baker, 1999).

A problem of the existing biocompatible and bioresorbable magnesium alloys is that they corrode too fast for the most prospective implant applications such as stents (vessel supports), bone fixtures plates and screws. The corrosion leads to the loss of mechanical integrity of the implant. In the case of stents for example, the structural support and the integrity must be maintained for in general at least three months after implantation. The poor corrosion resistance of magnesium also hinders the applications in other fields, e.g. as an alternative for aluminium.

Concerning the mechanical properties it can be mentioned that the ductility is in particular of importance for implants like stents. A stent must be able to shrink and to expand upon implantation. Actually, in practise the elongation in tension should be at least 15% and preferably even more than 18%.

European patent application EP 1 338 293 A1 discloses a process for producing bioresorbable metallic implants, in particular implants made out of magnesium and zinc alloys. Said patent application discloses that material properties and the processing and utilization properties can be changed by combining process steps for adjusting the material properties and subsequent machining. More in particular said patent application discloses the production of thin-walled tubular implants, particularly blood vessel support stents, from bioresorbable magnesium and zinc alloys, which are readily deformable without the risk of fracture during implantation. This known process comprises the steps of producing a pin-shaped semi-finished product or feedstock from the bioresorbable metal by casting, a heat treatment followed by an extrusion step, and the production of the tubular implant. The purpose of the first steps, viz. the casting and heat treatment is to distribute the alloying components of magnesium or zinc homogeneously. It is inherent to the casting process that (solidification) voids are created during this process. A disadvantage of this known process is that the product made accordingly exhibits a non-uniform microstructure as a result of macro-segregation. As a consequence, the quality of the material inside the product or semi-finished product is less than at the more outward lying parts. To make high quality products only the outer part should be selected for further processing. A further disadvantage is that the casting process is limited to conventional alloys, more particularly the concentration of the alloying elements in casting alloys is physically (thermodynamically) limited.

United States patent application US2005/0079099 A1 discloses the chemical modification of magnesium alloys by using halides to adjust and improve the corrosion resistance, more in particular to obtain a corrosion resistance that allows application of magnesium alloys in permanent implants and prostheses. This patent application discloses the use of A1 (Aluminium) for modification of magnesium alloys containing alloying elements Li (Lithium), Ca (Calcium) and rare-earth metals. This known modification of magnesium alloys can be performed by diffusion alloying or melt alloying. Also this process exhibits the disadvantage of a non-uniform microstructure and a relatively large grain structure.

### Summary of the invention

It is an objective of the present invention to provide a process for producing a product based on magnesium alloys or alloy mixtures, which product has an improved integrity in terms of corrosion resistance and that has mechanical properties that satisfy the demands for high quality applications. More in particular it is an objective to provide a process for producing biodegradable implants with an improved integrity. This objective of the invention is obtained by a production of the feedstock, an intermediate product, by semi-solid processing.

It is another objective of the present invention to provide magnesium based products that do not dissolve too fast in a human or animal body and that have no or at least acceptable toxic effects and that have good mechanical properties. Or more specifically, a product with an appropriate corrosion resistance and a high ductility. This objective of the invention is obtained by a product made by the process according to the invention.

### Brief description of the figures

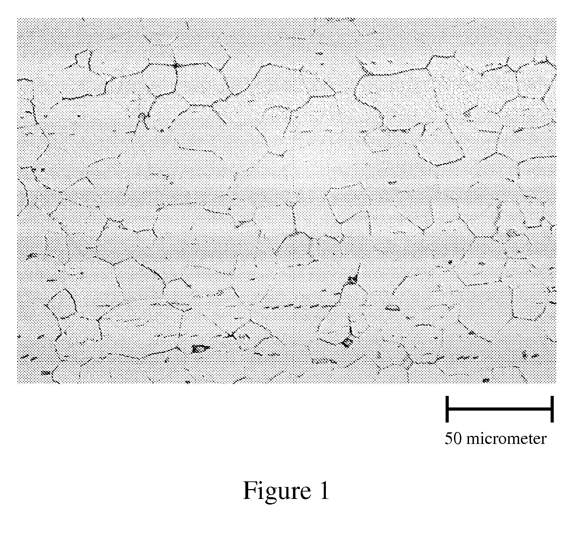

Fig. 1 contains a light optical microscopy picture of a product made out of hot extruded state-of-the -art magnesium alloy type AZ80.

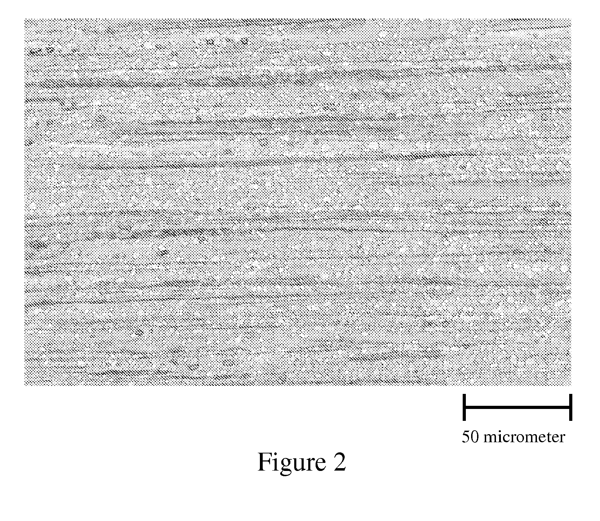

Fig. 2 contains a light optical microscopy picture of products made out of hot extruded semi-solid processed magnesium alloy type AZ80.
Fig. 3 shows an exemplary embodiment of the process according to the invention.

### Detailed description of the invention

The present invention is based upon the insight that the resistance to corrosion as well as the mechanical properties of magnesium based alloys and magnesium based alloy mixtures can be improved by combining a semi-solid processing with a subsequent thermo-mechanical treatment and an annealing step.

The present invention is also based on the insight that semi-solid processing allows the manufacturing of magnesium feedstock in which the concentration of alloying elements is not necessarily confined by the solution limits of these elements in the base element like it is in a casting process. Although this invention relates to magnesium as a base material, it is understood that the same principle holds for other base metals, like copper and aluminium.

Thirdly, the invention is based on the insight that even better corrosion resistance and more enhanced ductility with a low anisotropy between compression and tension can be obtained if the grain size of the magnesium based product is less than 5 micrometer, preferably less than 1 micrometer.

Finally it is recognized that the process is in particular advantageous for making biomedical implants like stents.

A large variety of magnesium alloys and mixtures of alloys can be used in the process according to the invention. The examples given below are just a few examples of specific alloys and feedstock.

**Table 1 Chemical composition of some specific experimental magnesium alloys and feedstock in wt%**

| alloy type | Al | Zn | Mn | Nd | Y | Ca | Sb | Mg |
|---|---|---|---|---|---|---|---|---|
| AZ80 | 8.0 | 0.5 | 0.2 | 0 | 0 | 0 | 0 | balanced |
| AE82 | 8.0 | 0.5 | 0.2 | 1.0 | 0.5 | 0 | 0 | balanced |
| AE82X | 8.0 | 0.5 | 0.2 | 1.0 | 0.5 | 0.8 | 0 | balanced |
| AE82xSb | 8.0 | 0.5 | 0.2 | 1.0 | 0.5 | 0.8 | 0.4 | balanced |
| ZE61 | 0 | 6.0 | 0.2 | 1.0 | 0 | 0 | 0 | balanced |
| ZE62 | 0 | 6.0 | 0.2 | 1.0 | 0.5 | 0 | 0 | balanced |
| ZE62X | 0 | 6.0 | 0.2 | 1.0 | 0.5 | 0.8 | 0 | balanced |
| ZE62XSb | 0 | 6.0 | 0.2 | 1.0 | 0.5 | 0.8 | 0.4 | balanced |

The amount of magnesium is balanced to obtain the 100 wt% for the alloy.

In addition to the elements mentioned in the table above, other alloying elements can be used. In particular rare-earth elements like Lanthanum, and other elements like Lithium, Manganese, Zirconium, Strontium, Silver, Copper, Zinc and Cadmium.

With respect to the compositions as given in the table and the description it will be appreciated that metallic alloys like magnesium alloys as used in practice may contain low levels of impurities that can not be removed from the alloy.

To secure the intrinsic corrosion resistance of the products made according to the process of the invention, a precursor or base material of a pre-defined chemical composition has to be manufactured. According to the present invention the precursor material may be either a magnesium alloy or a mixture of different magnesium alloys. Such alloys and mixtures of alloys with the desired composition can be manufactured by standard metallurgic methods know to those skilled in the art, like machining or grinding. Often stochiometric calculations are used to determine the composition. The corrosion potential of the alloying elements preferably is close to the corrosion potential of the primary magnesium phase because too much of a difference in corrosion potentials will cause an anodic-cathodic type of galvanic coupling, which will speed up the corrosion process, as with impurities like copper, nickel and iron. The precursor materials are processed into billets, granulates or other types of solid or hollow feedstock, including tubular shaped feedstock, for further processing. Examples of generally known manufacturing processes for feedstock are injection moulding, rheocasting and associated methods, continuous injection moulding, powder compaction, and metal injection moulding. Processing conditions and the type of manufacturing technique determine the (micro)structure and grain sizes of the feedstock.

According to the present invention the feedstock is manufactured with a semi-solid processing technique. Semi-solid processing allows the use a mixture of different precursors and base material. This results in a compound or blend with a specific composition that cannot be realised in melt processes like casting because such a material would liquate in the melt. Although novel types of alloys can be manufactured by semi-solid processing, this process is also advantageous for conventional alloys because the semi-solid processing allows the manufacturing of alloys with smaller grain size than is possible with other processing techniques.

A semi-solid processing technique is a technique where a mixture of a solid and a liquid is present. It is typically performed at a temperature that lies between the solidification temperature and the melting temperature of the material. An example of these techniques is thixomoulding, in which the viscous semi-solid is processed to obtain a feedstock by injection moulding. Another example is continuous extrusion of thixotropic magnesium, a process in which the magnesium or magnesium alloy emanates continuously from the extrusion apparatus. Typical for thixomoulding is the high speed with which the slurry is pushed into the mould. Semi-solid processing results in a unique (micro)structure that is uniform throughout the product. In addition the solidification voids are at a much lower level than in products made by casting.

One of the favourable properties of alloys and alloy mixtures made by semi-solid processing is that the concentration of the alloying elements is not limited by the solution limits of the elements in the base element. This effect of semi-solid processing is known for several elements, not only for magnesium but also for elements like aluminium and copper. Due to the fact that the concentration of the elements is not limited by the thermodynamically determined solution limits as given in phase-diagrams, a better tuning of the concentration of these elements is possible with regard to the properties that are required, like low corrosion rate and good mechanical properties. For biomedical applications like different types of stents (cardiovascular, urethral / prostatic, pancreatic) and bone plates, screws, foam structures to allow tissue growth inside the stent, the alloying elements can be chosen so that the stent has a high corrosion resistance in the human body, is ductile and that it causes no health problems. The specific tuning of the composition opens the way to use magnesium alloy for specific sensing purpose, for instance as an indicator for the integrity of an implant.

To give the above mentioned precursor or feedstock material a favourable strength and toughness the material has to be treated by at least one thermo-mechanical operation. Such an operation results in a re-crystallised (ultra)-fine microstructure of grain size preferably below 5 micrometer and even more preferably below 1 micrometer. As a consequence, the materials are likely to have a non-critical (reduced) release of larger particles and a low anisotropy between compression and tension. Examples of such thermo-mechanical operations are direct hot extrusion, hydrostatic extrusion, indirect hot extrusion, thixo-extrusion, conform extrusion, equal channel angular pressing and backward can extrusion.

After the thermo-mechanical step, annealing of the semi-finished product should take place for stress relieving and reconstructing the grain structure. It is appreciated that the process according to the invention may include additional thermal treatments to tune the mechanical response and the corrosion behaviour of the material. Such treatments are not specified here but may be included between or after each of the operations until a final product with the desired properties and shape is obtained.

Finally, the shape and the dimensions of the tubular product usually have to be adapted to the specifications of the product. Examples of such shaping operations are ironing, tube sinking or tube drawing, and fixed, moving or floating plug drawing.

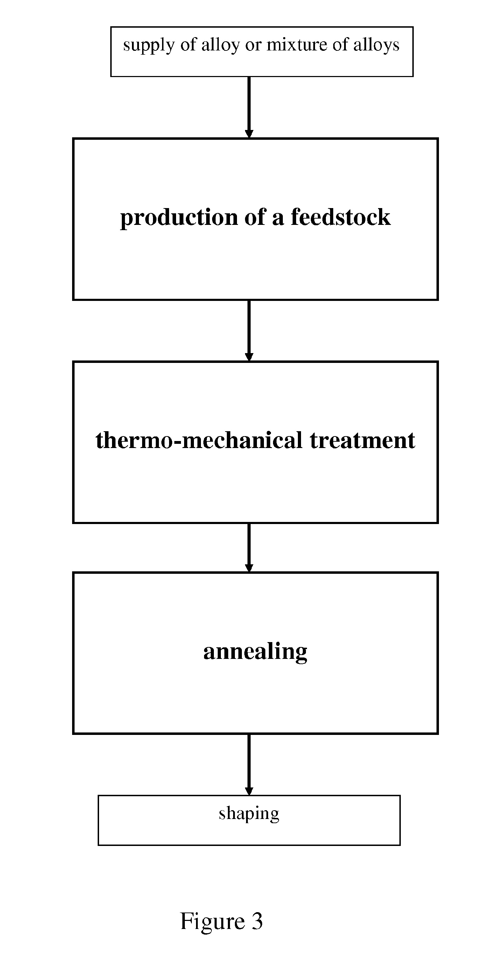

Figure 3 illustrates an examplary enbodiment of the process. It will be appreciated that the process may contain additional processing steps either before, in between or after the steps shown.

In conventional alloying often zirconium is used as a grain refiner. It has surprisingly been found that the desired grain size can be achieved without the addition of zirconium. This finding is very favourable for application in the human and animal body because of the alleged toxicity of zirconium.

Different rare-earth elements or combinations of rare-earth elements are used as alloying elements to improve the properties of magnesium. However, many of these elements, like for instance cerium, are known to be toxic. Therefore it is preferred to use for instance pure neodymium an element that is also used in medicine as a blood-thinning agent.

Also the use of Lanthanum (La) has shown to be advantageous for realizing a product with good properties.

More generally, the process according to the invention allows the fabrication of magnesium based products with good resistivity against corrosion and good mechanical properties while having no or at least low toxic or allergic effect.

### EXAMPLE

### AZ80 with 8.0 wt% Al, 0.5 wt% Zn and 0.2 wt% Mn.

The semi-solid state is achieved by heating chips and turnings in the range between 560 and 600 deg.C under an argon atmosphere during 1 to 10 minutes (hold time). A cooling rate between 1 and 2 deg.C per second was achieved by circulating liquid nitrogen in the mould through cooling channels. The smallest grain sizes were obtained at the shortest hold time and the fastest cooling rate.
The semi-solid processed material, viz. the feedstock was extruded at 375 deg.C at an area reduction ratio of R = 25. The grain is reduced from 16 micrometer to less than 3 micrometer after thermo-mechanical process of extrusion.
Next an annealing treatment was applied at 330 deg.C for 2 hours.
The corrosion rates were measured in a simulated body fluid at 37 deg.C. For the conventional AZ80 alloy the average corrosion rate of four experiments was 0.54 mm per year, whereas the rate for the material produced with the process according to the invention this rate was 0.35 mm per year.
The yield strength of the semi-solid processed magnesium alloy was 204 MPa, the ultimate tensile strength 314 MPa and the percentage elongation at fracture was improved up to 17.7%.

Further specific embodiments of the invention can be found in the addition of other materials than alloying elements. A material like hydroxyapatite for instance can be added to promote the healing of tissue surrounding the implant.

It is noted that any terms used in this document should not be construed so as to limit the scope of the present invention. In particular, the words "comprise(s)" and "comprising" are not meant to exclude any elements not specifically stated.

## Claims

**1.** A process for producing a magnesium based product, comprising
- the production of a feedstock out of a magnesium based alloy or a mixture of magnesium based alloys,
- a thermo-mechanical treatment of the feedstock, and
- an annealing step,
**characterized in that** the feedstock is produced by semi-solid processing.

**2.** A process according to claim 1, wherein the product is a tubular product.

**3.** A process according to claim 1 or claim 2, wherein the product is an implant.

**4.** A process according to any of the preceding claims, wherein the thermo-mechanical treatment is an extrusion process.

**5.** A process according to any of the preceding claims, wherein the semi-solid processing is a continuous process.

**6.** A process according to any of the preceding claims, wherein the process also comprises a shaping step after the thermo-mechanical treatment.

**7.** A process according to any of the preceding claims wherein the shaping is performed by tube sinking or drawing.

**8.** A product produced by the process according to any of the preceding claims.

**9.** A product according to claim 8, comprising Aluminium.

**10.** A product according to claim 8 or claim 9, comprising Zinc.

**11.** A product according to any of the claims 8 to 10, comprising Neodymium.

**12.** A product produced by the process according to any of the claims 1 to 7 wherein the product has a the grain size less than 5 micrometer, preferably less than 1 micrometer.

**12.** A product according to any of the claims 8 to 12 wherein the product is an implant.

**13.** A product according to claim 12 wherein the product is a stent.
